(19) Europäisches Patentamt
European Patent Office
Office européen des brevets

(11) Publication number : **0 568 307 A1**

(12) **EUROPEAN PATENT APPLICATION**

(21) Application number : **93303264.1**

(51) Int. Cl.$^5$ : **A61K 7/48,** A61K 47/12

(22) Date of filing : **27.04.93**

(30) Priority : **29.04.92 GB 9209238**

(43) Date of publication of application :
**03.11.93 Bulletin 93/44**

(84) Designated Contracting States :
**AT BE CH DE DK ES FR GB GR IE IT LI NL PT SE**

(71) Applicant : **UNILEVER PLC**
**Unilever House Blackfriars**
**London EC4P 4BQ (GB)**
(84) **GB IE**

(71) Applicant : **UNILEVER N.V.**
**Weena 455**
**NL-3013 AL Rotterdam (NL)**
(84) **BE CH DE DK ES FR GR IT LI NL PT SE AT**

(72) Inventor : **Evans, Brian**
**1 Raby Grove, Bebington**
**Wirral, Merseyside L63 5PX (GB)**
Inventor : **Morris, Christine**
**31 St James Avenue**
**Upton by Chester, Cheshire CH2 1NB (GB)**
Inventor : **Hagan, Desmond Bernard**
**35 Hookstone Drive**
**Little Sutton, South Wirral L66 4TE (GB)**

(74) Representative : **Tonge, Robert James et al**
**UNILEVER PLC Patent Division Colworth House Sharnbrook**
**Bedford MK44 1LQ (GB)**

(54) **Cosmetic composition containing alpha hydroxy carboxylic acids as preservatives.**

(57) The use of at least 0.0001% by weight based on the total composition of an hydroxy organic acid having the structure (1) :

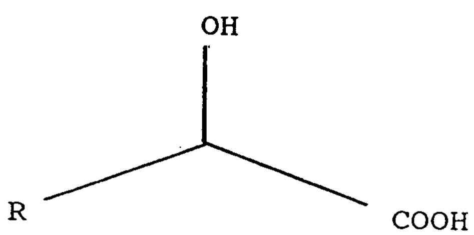

(1)

where

R is chosen from $C_xH_y$ and $C_xH_yOZ$,
x is an integer of from 2 to 16,
y is an integer of from 5 to 53,
Z is chosen from -H,R' and $(CH_2)_n$ OR',
R' is chosen from $C_aH_b$ and $(CH_2)_n$ $OC_aH_b$,
a is an integer of from 1 to 4,
b is an integer of from 2 to 9,
n is an integer of from 1 to 6.

and mixtures thereof ;
as an inhibitor of microbial spoilage, together with a major amount of a cosmetically acceptable vehicle, in a composition intended for topical application to the human body.

**EP 0 568 307 A1**

## Field of Invention

The invention relates to the use of selected hydroxy organic acids as preservatives in compositions, particularly compositions for topical application to the human body, which would otherwise be subject to microbial spoilage.

## Background to the Invention

Compositions that deteriorate or spoil as a result of the growth of microorganisms can have a very short shelf life, that is the period during which they can usefully be employed, unless they are refrigerated, frozen or sterilised within a sealed container. For compositions which are intended for occasional or regular use by the consumer, for example as beauty products or cleansing products for the skin, hair or teeth, it is inconvenient to maintain them under refrigerated conditions, and normally impossible to prevent contamination by adventitious microorganisms, particularly yeast, moulds and bacteria, of an otherwise sterile product, once the packaging that maintains sterility is opened.

In order to prevent microbial spoilage of such products, it has hitherto been necessary to incorporate a preservative chemical, such as 2-bromo-2-nitropropane-1,3-diol or methyl paraben, the level at which such preservatives are used being determined by the temperature at which the product is normally stored prior to each use and dependent also on the intrinsic properties of the composition itself in the provision of conditions that support the proliferation of microorganisms. In other words, high levels of preservative are necessary where the product is to be stored in a warm place or in tropical or sub-tropical conditions, or where the composition provides an excellent growth medium for adventitious microorganisms.

As research into the merits or demerits of using conventional preservative chemicals progresses, there is an increasing realisation that some of these chemicals can themselves deteriorate on storage, so that their preservative benefits diminish or are lost, or they can decompose in the product with the formation of bi-products that can be harmful to health. As an example, 2-bromo-2-nitropropane-1,3-diol has been shown under certain conditions to give rise to N-nitrosamines species which are alleged to be carcinogenic. Conventional preservatives can also be toxic in their own right unless used at very low levels at which their ability to prevent microbial spoilage is hardly adequate.

In view of the doubts and suspicions over the use of conventional chemical preservatives, there is an increasing demand by the consumer to provide products that are free from conventional preservatives, yet which are not prone to microbial spoilage. In order to meet this need, we have screened compounds that are believed to be totally safe at normal levels of use for their ability to prevent proliferation of microorganisms, many also having proven physiological benefits to the consumer.

As a result of this work, we have now identified a class of hydroxyorganic acids that meets this dual requirement of providing an in-use benefit to the consumer, while acting as an inhibitor of microbial spoilage.

We have also discovered that the preservative properties of these hydroxyorganic acids in terms of their ability to inhibit the proliferation of microorganisms, can be further enhanced to an unexpected extent by employing them together with sub-optimal concentrations of conventional chemical preservatives. Thus, conventional chemical preservatives, whose normal effective level of use has given rise to safety problems, can now be employed at lower than usual and hence safe levels in the presence of one or more of these hydroxyorganic acids.

## Definition of the Invention

Accordingly, the invention provides for the use of at least 0.0001% by weight based on the total composition of an hydroxy organic acid having the structure (1):

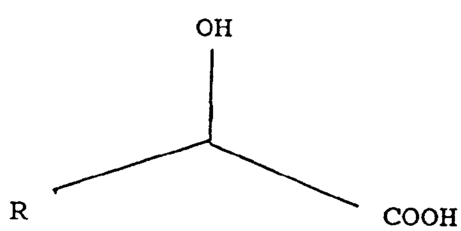

(1)

where

R is chosen from $C_xH_y$ and $C_xH_yOZ$
x is an integer of from 3 to 16,
y is an integer of from 7 to 33,
Z is chosen from -H,R' and $(CH_2)_nOR'$,
R' is chosen from $C_aH_b$ and $(CH_2)_nOC_aH_b$,
a is an integer of from 1 to 4,
b is an integer of from 2 to 9,
n is an integer of from 1 to 6.

and mixtures thereof;
as an inhibitor of microbial spoilage, in a composition intended for topical application to the human body. The composition will generally contain a major amount (i.e. over 50% by weight) of a cosmetically acceptable vehicle.

Disclosure of the Invention

The invention is accordingly concerned with the use of hydroxyorganic acids having the structure (1) as defined above in preventing microbial spoilage in otherwise perishable products, particularly compositions for topical human use.

Examples of 2-hydroxyalkanoic acids having the structure (1) include:
2-hydroxy-n-butanoic acid
2-hydroxy-n-pentanoic acid
2-hydroxy-n-hexanoic acid
2-hydroxy-n-octanoic acid
2-hydroxy-n-decanoic acid
2-hydroxy-n-dodecanoic acid
2-hydroxy-n-tetradecanoic acid and
2-hydroxy-n-hexadecanoic acid.

Examples of 2-hydroxyalkenoic acids having the structure (1) include:
2-hydroxy-4-hexenoic acid
2-hydroxy-4-octenoic acid
2-hydroxy-4-decenoic acid
2-hydroxy-4-dodecenoic acid
2-hydroxy-4-tetradecenoic acid, and
2-hydroxy-4-hexadecenoic acid.

Examples of di-unsaturated 2-hydroxyalkdienoic acids, having the structure (1) include:
2-hydroxy-4,7-octadienoic acid
2-hydroxy-4,9-decadienoic acid
2-hydroxy-4,10-undecadienoic acid
2-hydroxy-4,11-dodecadienoic acid
2-hydroxy-4,13-tetradecadienoic acid, and
2-hydroxy-4,15-hexadecadienoic acid.

Examples of mono unsaturated oxa-2-hydroxyalkenoic acids include:
2-hydroxy-6-oxa-4-heptenoic acid
2-hydroxy-7-oxa-4-octenoic acid
2-hydroxy-9-oxa-4-decenoic acid
2-hydroxy-9-oxa-4-undecenoic acid
2-hydroxy-11-oxa-4-dodecenoic acid
2-hydroxy-13-oxa-4-tetradecenoic acid
2-hydroxy-13-oxa-4-pentadecenoic acid
2-hydroxy-14-oxa-4-pentadecenoic acid
2-hydroxy-13-oxa-14-methyl-4-pentadecenoic acid
2-hydroxy-11,14-dioxa-4-pentadecenoic acid
2-hydroxy-13-oxa-4-hexadecenoic acid
2-hydroxy-14-oxa-4-hexadecenoic acid, and
2-hydroxy-15-oxa-4-hexadecenoic acid.

It is to be understood that the above examples of 2-hydroxyalkanoic acids and 2-hydroxyalkenoic acids are merely illustrative of the many acids covered by the generic structure (1) given hereinbefore.

The amount of the hydroxyorganic acid present in the composition for use in accordance with the invention

is from 0.0001 to 10%, preferably from 0.1 to 5% by weight.

The Cosmetically Acceptable Vehicle

The composition for use according to the invention also comprises a cosmetically acceptable vehicle to act as a dilutant, dispersant or carrier for the hydroxyorganic acid and other materials present in the composition, so as to facilitate their distribution when the composition is applied to the human body surface, particularly to the skin or hair.

Vehicles other than water can include liquid or solid emollients, solvents, humectants, thickeners and powders. Examples of each of these types of vehicle, which can be used singly or as mixtures of one or more vehicles, are as follows:

Emollients, such as stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rapeseed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate;

Propellants, such as propane, butane, isobutane, dimethyl ether, carbon dioxide, nitrous oxide;

Solvents, such as ethyl alcohol, methylene chloride, isopropanol, acetone, ethylene glycol monoethyl ether, diethylene glycol monobutyl ether, diethylene glycol monoethyl ether, dimethyl sulphoxide, dimethyl formamide, tetrahydrofuran;

Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The cosmetically acceptable vehicle will usually form from 10 to 99.9%, preferably from 50 to 99% by weight of the emulsion, and can, in the absence of other cosmetic adjuncts, form the balance of the emulsion.

pH

The composition according to the invention should preferably have a pH value not exceeding pH 7. Ideally, the pH value of the composition is from pH 4 to pH 7.

Adjustment of pH can be achieved by addition of a pH adjustant as conventionally used in the cosmetics art.

Conventional Chemical Preservatives

The composition for use according to the invention can optionally comprise one or more conventional chemical preservatives, preferably at a concentration that is lower than their normal level of use. Accordingly, these conventional chemical preservatives that may, when used alone, give rise to safety problem, when employed in sufficient quantity to prevent microbial spoilage, can in the presence of one or more hydroxyorganic acids, as herein defined, be effective as preservatives as sub-optional levels of use. Moreover, when the hydroxyorganic acids are employed together with one or more conventional chemical preservatives, then the effectiveness of such mixtures in preventing microbial spoilage can be unexpectedly high.

Examples of chemical preservatives that can be employed in this way include:

formaldehyde
2-bromo-2-nitropropane-1,3-diol: (e.g. Bronopol or Myacide BT)
methyl paraben: (e.g. Nipagin M)
propyl paraben: (e.g. Nipasol M)
butyl paraben: (e.g. Nipabutyl)
imidazolidinyl urea: (e.g. Germall 115)
diazolidinyl urea: (e.g. Germall II)
sorbic acid/potassium sorbate
benzoic acid/sodium benzoate
salicylic acid/sodium salicylate

phenoxyethanol
phenoxyethanol (80%) + dibromodiacyanobutane (20%): (e.g. Euxyl K400)
dimethylol dimethyl hydantoin: (e.g. Glydant)
Chloroacetamine
dehydroacetic acid
gluteraldehyde
methyl chloro isothiazolinone
phenyl ethyl acetate
1-(3-chloroallyl)-3,5,7-triaza-1-azonia adamantane chloride: (e.g. Quaternium 15)
sodium hydroxymethylglycinate
benzyl alcohol

Chemical preservatives can be employed individually or in mixtures, together with the hydroxy organic acid.

The amount of chemical preservative when employed will normally be at least 10% less than that normally necessary, in the absence of the hydroxyorganic acid, to inhibit microbial proliferation.

COSMETIC ADJUNCTS

The composition for use in accordance with the invention optionally can comprise cosmetic adjuncts, examples of which are given hereinafter, appropriate to the nature, function and intended use of the composition.

Organic Sunscreen Materials

The composition for use in accordance with the invention optionally can comprise one or more organic sunscreens, to provide protection from the harmful effects of excessive exposure to sunlight.

Examples of organic sunscreens, when required, include those set out in Table 1 below, and mixtures thereof.

Table 1

| CTFA Name | Trade Name | Supplier |
|---|---|---|
| Benzophenone-3 | UVINUL M-40 | BASF Chemical Co. |
| Benzophenone-4 | UVINUL MS-40 | BASF Chemical Co. |
| Benzophenone-8 | SPECRA-SORB UV-24 | American Cyanamide |
| DEA Methoxycinnamate | BERNEL HYDRO | Bernel Chemical |
| Ethyl dihydroxypropyl-PABA | AMERSCREEN P | Amerchol Corp. |
| Glyceryl PABA | NIPA G.M.P.A. | Nipa Labs. |
| Homosalate | KEMESTER HMS | Hunko Chemical |
| Methyl anthranilate | SUNAROME UVA | Felton Worldwide |
| Octocrylene | UVINUL N-539 | BASF Chemical Co. |
| Octyl dimethyl PABA | AMERSCOL | Amerschol Corp. |
| Octyl methoxycinnamate | PARSOL MCX | Givaudan |
| Octyl salicylate | SUNAROME WMO | Felton Worldwide |
| PABA | PABA | National Starch |
| 2-Phenylbenzimidazole-5-sulphonic acid | EUSOLEX 232 | EM Industries |
| TEA salicylate | SUNAROME W | Felton Worldwide |
| 3-(4-methylbenzylidene)-camphor | EUSOLEX 6300 | EM Industries |
| Benzophenone-1 | UVINUL 400 | BASF Chemical Co. |
| Benzophenone-2 | UVINUL D-50 | BASF Chemical Co. |
| Benzophenone-6 | UVINUL D-49 | BASF Chemical Co. |
| Benzophenone-12 | UVINUL 408 | BASF Chemical Co. |
| 4-Isopropyl dibenzoyl methane | EUSOLEX 8020 | EM Industries |
| Etocrylene | UVINUL N-35 | BASF Chemical Co. |

The composition of the invention can accordingly comprise from 0.1 to 20%, preferably from 1 to 10% by weight of organic sunscreen materials.

## Inorganic Sunscreens

The composition of the invention optionally can also comprise one or more inorganic sunscreens.

Currently, the most widely used inorganic sunscreen is ultrafine titanium dioxide, having an average particle size of from 1 to 100 nm, preferably from 10 to 40 nm, which offers both absorbance and reflectance of ultra violet light.

Examples of other inorganic sunscreens include zinc oxide, iron oxide, silica, such as fumed silica and boron nitride, each having an average particle size of from 1 to 100nm.

## Emulsion ingredients

A particularly convenient form of the composition according to the invention is an emulsion, in which case an oil or oily material will normally be present, together with an emulsifier to provide either a water-in-oil emulsion or an oil-in-water emulsion, depending largely on the average hydrophilic-lyophilic balance (HLB) of the

emulsifier employed.

Oil or oily material

The composition according to the invention can optionally comprise one or more oils or other materials having the properties of an oil.

Examples of suitable oils include mineral oil and vegetable oils, and oil materials, such as those already proposed herein as emollients. Other oils or oily materials include silicone oils, both volatile and non-volatile, such as polydimethyl siloxanes.

The oil or oily material, when present for the purposes for forming an emulsion, will normally form up to 90%, preferably from 10 to 80% by volume of the composition.

Emulsifier

The composition according to the invention can also optionally comprise one or more emulsifiers the choice of which will normally determine whether a water-in-oil or and oil-in-water emulsion is formed.

When a water-in-oil emulsion is required, the chosen emulsifier or emulsifiers should normally have an average HLB value of from 1 to 6. When an oil-in-water emulsion is required, a chosen emulsifier or emulsifiers should have an average HLB value of >6.

Examples of suitable emulsifiers are set below in Table 2 in which the chemical name of the emulsifiers is given together with an example of a trade name as commercially available, and the average HLB value.

Table 2

| Chemical Name of Emulsifier | Trade Name | HLB Value |
|---|---|---|
| Sorbitan trioleate | Arlacel 85 | 1.8 |
| Sorbitan tristearate | Span 65 | 2.1 |
| Glycerol monooleate | Aldo MD | 2.7 |
| Glycerol monostearate | Atmul 84S | 2.8 |
| Glycerol monolaurate | Aldo MC | 3.3 |
| Sorbitan sesquioleate | Arlacel 83 | 3.7 |
| Sorbitan monooleate | Arlacel 80 | 4.3 |
| Sorbitan monostearate | Span 60 | 4.7 |
| Poloxyethylene (2) stearyl ether | Brij 72 | 4.9 |
| Poloxyethylene sorbitol beeswax derivative | G-1702 | 5 |
| PEG 200 dilaurate | Emerest 2622 | 6.3 |
| Sorbitan monopalmitate | Arlacel 40 | 6.7 |
| Polyoxyethylene (3.5) nonyl phenol | Emulgen 903 | 7.8 |
| PEG 200 monostearate | Tegester PEG 200 MS | 8.5 |
| Sorbitan monolaurate | Arlacel 200 | 8.6 |
| PEG 400 dioleate | Tegester PEG 400-DO | 8.8 |
| Polyoxyethylene (5) monostearate | Ethofat 60-16 | 9.0 |
| Polyoxyethylene (4) sorbitan monostearate | Tween 61 | 9.6 |
| Polyoxyethylene (4) lauryl ether | Brij 30 | 9.7 |
| Polyoxyethylene (5) sorbitan monooleate | Tween 81 | 10.0 |
| PEG 300 monooleate | Neutronyx 834 | 10.4 |

| | | |
|---|---|---|
| Polyoxyethylene (20) sorbitan tristearate | Tween 65 | 10.5 |
| Polyoxyethylene (20) sorbitan trioleate | Tween 85 | 11.0 |
| Polyoxyethylene (8) monostearate | Myrj 45 | 11.1 |
| PEG 400 monooleate | Emerest 2646 | 11.7 |
| PEG 400 monostearate | Tegester PEG 400 | 11.9 |
| Polyoxyethylene 10 monooleate | Ethofat 0/20 | 12.2 |
| Polyoxyethylene (10) stearyl ether | Brij 76 | 12.4 |
| Polyoxyethylene (10) cetyl ether | Brij 56 | 12.9 |
| Polyoxyethylene (9.3) octyl phenol | Triton X-100 | 13.0 |
| Polyoxyethylene (4) sorbitan monolaurate | Tween 21 | 13.3 |
| PEG 600 monooleate | Emerest 2660 | 13.7 |
| PEG 1000 dilaurate | Kessco | 13.9 |
| Polyoxyethylene sorbitol lanolin derivative | G-1441 | 14.0 |
| Polyoxyethylene (12) lauryl ether | Ethosperse LA-12 | 14.4 |
| PEG 1500 dioleate | Pegosperse 1500 | 14.6 |
| Polyoxyethylene (14) laurate | Arosurf HFL-714 | 14.8 |
| Polyoxyethylene (20) sorbitan monostearate | Tween 60 | 14.9 |
| Polyoxyethylene 20 sorbitan monooleate | Tween 80 | 15.0 |
| Polyoxyethylene (20) stearate | Myrj 49 | 15.0 |
| Polyoxyethylene (20) stearyl ether | Brij 78 | 15.3 |

| | | |
|---|---|---|
| Polyoxyethylene (20) sorbitan monopalmitate | Tween 40 | 15.6 |
| Polyoxyethylene (20) cetyl ether | Brij 58 | 15.7 |
| Polyoxyethylene (25) oxypropylene monostearate | G-2162 | 16.0 |
| Polyoxyethylene (20) sorbitol monolaurate | Tween 20 | 16.7 |
| Polyoxyethylene (23) lauryl ether | Brij 35 | 16.9 |
| Polyoxyethylene (50) monostearate | Myrj 53 | 17.9 |
| PEG 4000 monostearate | Pegosperse 4000 MS | 18.7 |

The foregoing list of emulsifiers is not intended to be limiting and merely exemplifies selected emulsifiers which are suitable for use in accordance with the invention.

It is to be understood that two or more emulsifiers can be employed if desired.

The amount of emulsifier or mixtures thereof, to be incorporated in the composition of the invention, when appropriate is from 1 to 50%, preferably from 2 to 20% and most preferably from 2 to 10% by weight of the composition.

Water

The composition of the invention can also comprise water, usually up to 80%, preferably from 5 to 80% by volume.

Silicone Surfactant

The composition of the invention can also optionally comprise a high molecular weight silicone surfactant which can also act as an emulsifier, in place of or in addition to the optional emulsifier(s) already mentioned.

The silicone surfactant is a high molecular weight polymer of dimethyl polysiloxane with polyoxyethylene and/or polyoxypropylene side chains having a molecular weight of from 10,000 to 50,000 and having the structure:

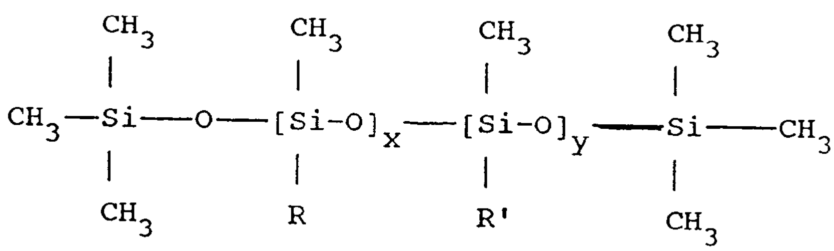

where

R is a $C_{1-18}$ alkyl group and

R' is a polyether group having the structure:

$$CH_3H_5O(C_2H_4O)_a(CH_3C_2H_3O)_bR''$$

where R" is H or a $C_{1-18}$ alkyl group,
a has a value of from 9 to 115,
b has a value of from 0 to 50,
x has a value of from 133 to 673,
y has a value of from 25 to 0.25.

Preferably, the dimethyl polysiloxane polymer is one in which:
a has a value of from 10 to 114
b has a value of from 0 to 49
x has a value of from 388 to 402
y has a value of from 15 to 0.75
one of groups R' and R" being lauryl, and the other having a molcular weight of from 1000 to 5000.

A particularly preferred dimethyl polysiloxane polymer is one in which:
a has the value 14
b has the value 13
x has the value 249
y has the value 1.25

The dimethyl polysiloxane polymer is conveniently provided as a dispersion in a volatile siloxane, the dispersion comprising, for example, from 1 to 20% by volume of the polymer and from 80 to 99% by volume of the volatile siloxane. Ideally, the dispersion consists of a 10% by volume of the polymer dispersed in the volatile siloxane.

Examples of the volatile siloxanes in which the polysiloxane polymer can be dispersed include polydimethyl siloxane (pentamer and/or hexamer).

A particularly preferred silicone surfactant is cyclomethicone and dimethicone copolyol, such as DC 3225C Formulation Aid available from DOW CORNING. Another is laurylmethicone copolyol, such as DC Q2-5200, also available from Dow Corning.

The amount of silicone surfactant, when present in the composition will normally be up to 25%, preferably from 0.5 to 15% by weight of the emulsion.

Surfactant

The composition according to the invention can also optionally comprise a surfactant chosen from anionic, nonionic or amphoteric surfactant or mixtures thereof, particularly when the composition is intended for shampooing the hair or for use when bathing or in the shower.

Suitable anionic surfactants for this purpose are the alkyl sulphates, alkyl ether sulphates, alkaryl sulphonates, alkyl succinates, alkyl sulphosuccinates, N-alkoyl sarcosinates, and alpha-olefin sulphonates, expecially their sodium, magnesium, ammonium and mono-, di- and triethanolamine salts. The alkyl groups generally contain from 8 to 18 carbon atoms and may be unsaturated. The alkyl ether sulphates may contain from one to 10 ethylene oxide or propylene oxide units per molecule, and preferably contain 2 to 3 ethylene oxide units per molecule.

Examples of suitable anionic surfactants include sodium lauryl sulphate, sodium oleyl succinate, ammonium lauryl sulphosuccinate, ammonium lauryl sulphate, sodium dodecylbenzene sulphonate, triethanolamine dodecylbenzene sulphonate and sodium N-lauryl sarcosinate. The most preferred anionic surfactants are sodium lauryl sulphate, sodium lauryl ether sulphate 2EO and 3EO, ammonium lauryl sulphate and ammonium lauryl ether sulphate 1EO, 2EO and 3EO.

The nonionic surfactants suitable for optional use in the composition of the invention may include condensation products of aliphatic ($C_8$-$C_{18}$) primary or secondary linear or branched chain alcohols or phenols with alkylene oxides, usually ethylene oxide and generally 6-30 EO.

Other suitable nonionics include mono or di alkyl alkanolamides or alkyl polyglucosides. Examples include coco mono or diethanolamide, coco mono isopropanolamide, and coco di glucoside.

The amphoteric surfactants suitable for optional use in the composition of the invention may include alkyl amine oxides, alkyl etaines, alkyl amidopropyl betaines, alkyl mono- or dialkanolamides, alkyl sulphobetaines, alkyl glycinates and alkyl carboxyglycinates, wherein the alkyl groups have from 8 to 18 carbon atoms. Examples include lauryl amine oxide, cocomonoethanolamide, cocodiethanolamide, cocomidopropyl betaine, cocodimethyl sulphopropyl betain and preferably cocobetaine.

The surfactants when present in the composition of the invention form from 2 to 40% by weight, and preferably from 5 to 30% by weight.

Other Cosmetic Adjuncts

Examples of conventional adjuncts which can optionally be employed include antioxidants, such butyl hydroxy toluene; humectants, such as glycerol, sorbitol, 2-pyrrolidone-5-carboxylate, dibutylphthalate, gelatin, polyethylene glycol, such as PEG 200-600; buffers, such as lactic acid together with a base such as triethanolamine or sodium hydroxide; waxes, such as beeswax, ozokerite wax, paraffin wax: plant extracts, such as Aloe vera, cornflower, witch hazel, elderflower, cucumber; thickeners; activity enhancers; colourants; and perfumes. Cosmetic adjuncts can form the balance of the composition.

Use of the hydroxyorganic acid as a preservative

The hydroxyorganic acid, as herein defined, or mixtures thereof can be used to preserve a wide variety of composition intended for topical application to the human body. These compositions can, for example, include skin creams, lotion, milks and powders, skin cleansing products, hair treatment products, such as shampoos, conditioners, styling aids, and dental products such as toothpaste and mouthwash.

PRODUCT FORM AND PACKAGING

The topical compositions in which the hydroxyorganic acid is used as a preservative can be formulated as a liquid, gel paste or powder, or as a lotion having a viscosity of from 4,000 to 10,000 mPas, a fluid cream having a viscosity of from 10,000 to 20,000 mPas or a cream having a viscosity of from 20,000 to 100,000 mPas, or above. The composition can be packaged in a suitable container to suit its viscosity and intended use by the consumer.

For example, a liquid, gel, lotion or fluid cream can be packaged in a bottle or a roll-ball applicator or a propellant-driven aerosol device or a container fitted with a pump suitable for finger operation. As a shampoo or hair conditioner, this composition can be packaged in a bottle or sachet. When the composition is a cream or paste, it can simply be stored in a non-deformable bottle or squeeze container, such as a tube or a lidded jar.

The invention accordingly also provides a closed container containing a cosmetically acceptable composition as herein defined.

Process

The invention also provides a process for preparing a preserved composition which comprises the steps of distributing an effective amount of hydroxyorganic as herein defined, in a cosmetically acceptable carrier, and adjusting the pH of the composition so formed, as necessary, to a value not exceeding pH 7.

Challenge Test Procedure for evaluating antimicrobial activity of preservatives in composition of the invention

A test was devised to assess the ability of selected hydroxyorganic acids to preserve, against microbial spoilage, compositions according to the invention and, by way of comparison, others containing conventional chemical preservative substances.

This test involved incubating under standard conditions test compositions which have been inoculated with a selected bacterium, yeast or a mixture of moulds. Each test composition was repeatedly inoculated at regular intervals ("challenged"), until the surviving micro-organisms from the inoculation showed an increase above a standard level, at which point, the preservative systems under test was judged to have failed. Accordingly, the higher the number of "challenges", the better is the preservative. substance in preventing or at least delaying microbial spoilage.

Materials

A series of compositions containing hydroxyorganic acids chosen from those in accordance with the invention and/or conventional chemical preservatives was prepared.

The micro-organisms selected for this test were:

i) Pseudomonas cepacia - a gram-negative bacterium,

ii) Candida parapsilosis - a yeast, and

iii) Aspergillus niger & Penicillium sp., - a mixture of moulds.

The formulations used in the Challenge Test were creams, lotions and shampoos as set out in the Results section below.

Method

The method of testing was as follows:

i) Introduce aseptically 99 ml of test composition into a 250 ml flask and add 1 ml of inoculum of one of the test organisms (see above), to provide a dilution of $10^6$ cells/ml in the composition.

ii) mix and incubate at 28°C for 24 hours.

iii) remove 1 ml of incubated composition from the flask and add to it 9 ml of peptone water/Tween 80 as diluent and mix thoroughly.

iv) place 1 ml of diluted sample into a petri dish and add warm, molten agar (40°C) and mix to distribute culture uniformly and allow to set.

v) incubate plates for 3 days at 28°C and then count colonies and note log reduction of count (due to the presence of the preservative in the original culture composition).

The inoculation of the organisms originally introduced into each composition is then repeated at 24 hour intervals, following removal of the first total viable count sample, the plating out and counting procedure being repeated on each occasion. The test is terminated when the samples show a contamination level greater than $10^2$ counts per ml on 2 consecutive days. This point is regarded as the failure point for the composition under test.

The greater the number of inoculations of contaminant organisms before this level of contamination is established in the composition, the better the preservative system.

The above procedure applies to the inoculation of the bacterium and the yeast, and this procedure was varied slightly with the mixed culture of moulds as follows:

i) two samples of each test composition (9.9 ml and 9 ml) were transferred to sterile flasks and 0.1 ml or 1 ml of a mould spore suspension (approximately $10^8$ spores per ml) was introduced into the respective samples and thoroughly mixed. The inoculated samples were incubated at 28°C for up to 28 days with 1 ml samples being aseptically removed for the total viable count as outlined above using Sabouraud's dextrose agar after 1, 7, and 28 days.

ii) "Low risk" compositions eliminate mould spores in 1 to 2 days, "medium risk" compositions take 7 to 14 days to eliminate the spores and the "high risk" compositions still have surviving mould spores after 14 days incubation.

Results

A series of ten experiments was conducted to study the benefits of using the hydroxyorganic acids, some compared with conventional chemical preservatives, in preventing microbial spoilage.

Experimental Series I

This series was designed to compare the ability of selected hydroxyorganic acids to inhibit microbial growth in cream compositions containing the conventional chemical preservative methyl paraben.

The formulations used are set out in Table 3 below.

Table 3

| Ingredient | IA | IB | IC | ID |
|---|---|---|---|---|
| 2-Hydroxy octanoic acid | 1.00 | - | - | - |
| 2-hydroxy-12-oxa-4-tetradecenoic acid | - | 1.00 | - | - |
| 2-hydroxy-10-undecenoic acid | - | - | 1.00 | - |
| Lactic acid | 5.00 | 5.00 | 5.00 | 5.00 |
| Butane-1,3-diol | 10.00 | 10.00 | 10.00 | 10.00 |
| Sodium hydroxide | 7.40 | 8.50 | 6.75 | 7.20 |
| Sodium chloride | 2.00 | 2.00 | 2.00 | 2.00 |
| Silicone oil | 20.20 | 20.20 | 20.20 | 20.20 |
| Nipagin M | 0.20 | 0.20 | 0.20 | 0.20 |
| Nipasol M | 0.10 | 0.10 | 0.10 | 0.10 |
| Tiona AG | 0.20 | 0.20 | 0.20 | 0.20 |
| Petroleum jelly | 0.50 | 0.50 | 0.50 | 0.50 |
| Mineral oil | 1.50 | 1.50 | 1.50 | 1.50 |
| Perfume | 0.15 | 0.15 | 0.15 | 0.15 |
| Water (demin.) | 51.75 | 50.65 | 52.40 | 52.95 |
| | 100.00 | 100.00 | 100.00 | 100.00 |
| pH | 4.0 | 4.0 | 4.0 | 4.0 |

The results are shown in Table 4 below:

Table 4

| Formulation Code | Number of inoculations to failure | |
|---|---|---|
| | Bacteria | Yeast |
| IA | >46 | >46 |
| IB | >46 | >46 |
| IC | >46 | >46 |
| ID (control) | >46 | 34 |

These results indicated that each of the cream formulations containing 1% by weight of an hydroxyorganic acid (i.e. Formulations IA, IB and IC) showed superior preservative properties for both bacteria and yeast, compared with the control (Formulation ID) containing the conventional preservatives lactic acid and parabens, which failed the anti-yeast challenges even at pH4.0.

Experimental Series II

This series was designed to compare the ability of selected hydroxyorganic acids to inhibit microbial growth in cream compositions otherwise containing no conventional chemical preservatives.

The formulations used are set out in Table 5 below:

Table 5

| Ingredient | IIA | IIB | IIC | IID |
|---|---|---|---|---|
| 2-hydroxy octanoic acid | 1.00 | - | - | - |
| 2-Hydroxy-4-dodecanoic acid | - | - | 1.00 | - |
| 2-Hydroxy-13-oxa-4-tetradecenoic acid | - | - | - | 1.00 |
| Sodium hydroxide | 0.80 | - | 1.25 | 7.00 |
| Butane-1,3-diol | 10.00 | 10.00 | 10.00 | 10.00 |
| Sodium chloride | 2.00 | 2.00 | 2.00 | 2.00 |
| Silicone oil | 20.20 | 20.20 | 20.20 | 20.20 |
| Tiona AG | 0.20 | 0.20 | 0.20 | 0.20 |
| Petroleum jelly | 0.50 | 0.50 | 0.50 | 0.50 |
| Mineral oil | 1.50 | 1.50 | 1.50 | 1.50 |
| Water (demin.) | 63.80 | 65.60 | 63.20 | 57.60 |
| Perfume | - | - | 0.15 | - |
| | 100.00 | 100.00 | 100.00 | 100.00 |
| pH | 5.5 | 5.7 | 5.8 | 5.4 |

The results are shown in Table 6 below:

Table 6

| Formulation Code | Number of inoculations to failure | | Mould challenge | |
|---|---|---|---|---|
| | Bacteria | Yeasts | 1% | 10% |
| IIA | >23 | >23 | - | + |
| IIB | 1 | 1 | +++ | ++++ |
| IIC | 21 | 1 | + | ++++ |
| IID | 5 | 3 | + | + |

Key to Mould Challenge (after injection of spores)

- \- No mould growth 1, 7, 14 or 28 days
- \+ Mould growth 1 day after; no growth after 7, 14 or 28 days
- ++ Mould growth 1 and 7 days after; no growth after 14 or 28 days
- +++ Mould growth 1, 7 and 14 days after; no growth after 28 days
- ++++ Mould growth 1, 7, 14 and 28 days after injection of spores.

These results indicate that when the pH value of the composition is raised from 4.0 (cf. Series I) to 5.5, then 2-hydroxy octanoic acid (formulation IIA), in the absence of other conventional preservatives provides adequate preservation. The unsaturated hydroxy acid in formulation IIC provided good preservation against bacteria. The 'oxa' hydroxy acid provided good preservation against the mould challenge.

Experimental Series III

This series was designed to compare the ability of selected hydroxy-organic acids to inhibit microbial growth in cream compositions as compared with similar creams containing lactic acid as a preservative, or no conventional chemical preservative.

The formulations and are set out in Table 7 below.

Table 7

| Ingredient | IIIA | IIIB | IIIC | IIID |
|---|---|---|---|---|
| Lactic acid | - | 1.0 | - | - |
| 2-Hydroxy hexadecanoic acid | - | - | 1.0 | - |
| 2-Hydroxy butanoic acid | - | - | - | 1.0 |
| Sodium hydroxide | 3.0 | 1.0 | 0.75 | 0.25 |
| Hydrochloric acid | - | - | - | - |
| Butaine-1,3-diol | 10.0 | 10.0 | 10.0 | 10.0 |
| Sodium chloride | 2.0 | 2.0 | 2.0 | 2.0 |
| Silicone oil, DC344 | 8.2 | 8.2 | 8.2 | 8.2 |
| Silicone oil, DC3225C | 12.0 | 12.0 | 12.0 | 12.0 |
| Tiona AG | 0.2 | 0.2 | 0.2 | 0.2 |
| Petroleum jelly | 0.5 | 0.5 | 0.5 | 0.5 |
| Mineral | 1.5 | 1.5 | 1.5 | 1.5 |
| Perfume | 0.15 | 0.15 | 0.15 | 0.15 |
| Water (demin.) | 62.45 | 63.45 | 63.70 | 64.20 |
| | 100.00 | 100.00 | 100.00 | 100.00 |
| pH | 5.5 | 5.5 | 5.5 | 5.5 |

The results are shown in Table 8 below:

Table 8

| Formulation Code | Number of inoculations of Bacteria to failure | Mould Challenge |
|---|---|---|
| IIIA (control) | 1 | ++++ |
| IIIB (control) | >23 | ++ |
| IIIC | 1 | + |
| IIID | 1 | ++++ |

Key to Mould challenge: see footnotes under Table 6.

These results show that lactic acid (formulation IIIB) a conventional antibacterial preservative, is a poor preservative against moulds, while 2-hydroxy butanoic acid (formulation IIID) is slightly better against moulds but ineffective against bacteria.

Experimental Series IV

This series was designed to compare the effectiveness of 2-hydroxy octanoic acid at different concentrations as a preservative in a water-in-oil cream at pH 5.5

The results are shown in Table 9 below.

Table 9

| Series IV | | | |
|---|---|---|---|
| 2-hydroxyoctanoic acid (% w/w) in water-in-oil cream | number of inoculations of bacteria to failure | mould challenge | |
| | | 1% | 10% |
| 0 | 4 | ++ | ++ |
| 0.1 | 4 | ++ | ++ |
| 0.25 | 4 | ++ | ++ |
| 0.5 | 20 | + | ++ |
| 0.75 | >20 | + | ++ |
| 1 | >20 | - | - |

These results show that 2-hydroxyoctanoic acid in a water-in-oil cream at pH 5.5. provides good preservative at 0.5% w/w and above. The best result was obtained using 1% 2-hydroxy octanoic acid.

Experimental Series V

This series was designed to compare a hydroxy organic acid with other materials as inhibitors of mould growth in cream compositions.

A base formulation used for all experiments in the series was an oil-in-water skin cream which contained:

| | |
|---|---|
| Butane-1,3-diol | 13.5 |
| Xanthan Gum (Rhodopol 23) | 0.5 |
| Ozokerite Wax | 2.56 |
| Glyceryl Monostearate | 3.84 |
| Hexadecanol | 0.6 |
| Titanium Dioxide | 0.15 |
| Volatile Silicone (DC345) | 7.6 |
| Ethoxylated Emulsifier (Brij 58) | 1.4 |
| Sodium Hydroxide (to pH 5.5) | qs |
| Demineralised Water | to 100 |

Various preservative materials were added to samples of this base formulation. The resulting compositions were subjected to the mould challenge test, as set out in Table 10 below.

Table 10

| Added preservative and concentration (% w/w) | No of inoculations to failure | Mould challenge 1% |
|---|---|---|
| None (control) | 1 | ++++ |
| 2-hydroxyoctanoic acid (1%) | >20 | + |
| Lauric acid (1%) | 1 | ++++ |
| Monolaurin (1%) | 1 | ++++ |
| Commercially available bacteriostat (0.3%) | 1 | ++++ |
| Combination of: Nipagen M 0.2%, Nipasol M 0.1%, Empanox BH7 0.05%, Bronopol 0.01% | >20 | + |

As shown by the above Table 10, 2-hydroxyoctanoic acid had a preservative action which matched the mixture of preservatives and outperformed several other materials. The commercially available bacteriostat was used at its manufacturer's recommended dosage.

## EXAMPLES

The invention is illustrated by the following examples; in each case, the pH value was adjusted to a value of from 5.0 - 5.5.

Examples 1 to 6 illustrate cream compositions according to the invention.

Example 1

| | % w/w |
|---|---|
| 2-hydroxy-n-octanoic acid | 1 |
| Silicone oil 200 cts | 7.5 |
| Glycerylmonostearate | 3 |
| Cetosteryl alcohol | 1.6 |
| Polyoxyethylene-(20)-cetyl alcohol | 1.4 |
| Xanthan gum | 0.5 |
| Parsol 1789 | 1.5 |
| Octyl methoxycinnate (PARSOL MCX) | 7 |
| Perfume | qs |
| Colour | qs |
| Water | to 100 |

This composition showed excellent antibacterial and antifungal properties when stored at 30°C.

Example 2

| | % w/w |
|---|---|
| 2-hydroxy-n-nonanoic acid | 2 |
| Decyloleate | 6 |
| Isopropylpalmitate | 6 |
| Polyoxyethylene-(20)-stearate | 0.85 |
| Sorbitan monostearate | 0.6 |
| Triethanolamine | 0.26 |
| Fatty acid esters | 0.2 |
| Carboxyvinylpolymer | 0.26 |
| Parsol 1789 | 2 |
| 2-phenyl-benzimidazole-S-sulphonic acid (EUSOLEX 232) | 4 |
| sodium benzoate | 0.5 |
| Perfume | qs |
| Colour | qs |
| Water | to 100 |

This composition showed excellent antibacterial properties when stored at 30°C.

Example 3

| | % w/w |
|---|---|
| 2-hydroxy-4-dodecenoic acid | 1 |
| Mineral oil | 20 |
| Microcrystalline wax | 5 |
| Sorbitan sesquioleate | 1.5 |
| Parsol 1789 | 0.4 |
| Oxybentone | 1 |
| Parsol MCX | 2 |
| Perfume | qs |
| Colour | qs |
| Water | to 100 |

This composition showever excellent antibacterial properties when stored at 30°C.

Example 4

| | % w/w |
|---|---|
| 2-hydroxy-13-oxa-4-tetradecenoic acid | 0.5 |
| POE*-(2)-stearyl alcohol | 4.8 |
| POE-(20)-stearyl alcohol | 1.2 |
| POP*-(15)-stearyl alcohol | 12.0 |
| Parsol 1789 | 3.0 |
| Parsol MCX | 6.0 |
| Octyl salicylate | 2.0 |
| Perfume | qs |
| Colour | qs |
| Water | to 100 |

* POE = polyoxyethylene

**POP = polyoxypropylene

This composition showed moderate antibacterial activity.

Example 5

| | % w/w |
|---|---|
| 2-hydroxy-n-butanoic acid | 1 |
| Mono, di, tri-($C_{12}$-$C_{14}$ tetraglycol ether)-O-phosphonic acid esters | 9 |
| Paraffin oil | 30 |
| Isopropylpalmitate | 6 |
| Silicone oil (20 CTS) | 1 |
| Lanolin oil | 1 |
| Cetyl alcohol | 2 |
| Parsol 1789 | 2 |
| Ultrafine $TiO_2$ | 2 |
| Perfume | qs |
| Colour | qs |
| Water | to 100 |

This composition showed excellent antifungal activity.

Example 6

| | % w/w |
|---|---|
| 2-hydroxyoctanoic acid | 1 |
| Silicone oil 200 cts | 7.5 |
| Glycerylmonostearate | 3 |
| Cetosteryl alcohol | 1.6 |
| Polyoxyethylene-(20)-cetyl alcohol | 1.4 |
| Xanthan gum | 0.5 |
| Pongamol | 1.5 |
| PARSOL MCX | 7 |
| Methylparabens | 0.1 |
| Propylparabens | 0.05 |
| Perfume | qs |
| Colour | qs |
| Water | to 100.00 |

This composition showed excellent antibacterial and antifungal properties. The concentration of both methyl parabens and propyl paraben were half those normally employed as preservatives.

Example 7

This example illustrates a water-in-oil skin cream according to the invention. The cream had the following formulation:

| | % w/w |
|---|---|
| 2-Hydroxy octanoic acid | 1 |
| Butane-1,3-diol | 10 |
| Sodium Chloride | 2 |
| Volatile Silicone Oil (DC344: DC3225C = 8.2:12.0) | 20.2 |
| Titanium Dioxide | 0.2 |
| Petroleum Jelly | 0.5 |
| Mineral Oil | 1.5 |
| Fragrance | 0.15 |
| Sodium Hydroxide (to pH 5.5) | qs |
| Demineralised Water | to 100 |

Example 8

This example illustrates a shampoo in accordance with the invention. The shampoo had the following formulation:

| | % w/w |
|---|---|
| 2-Hydroxy octanoic acid | 1 |
| Sodium lauryl ether sulphate | 11 |
| Lauryl dimethyl betaine | 2 |
| Coconut diethanolamide | 1 |
| Electrolyte (to adjust viscosity) | 1.3 |
| Citric Acid (to pH 6.0) | qs |
| Demineralised Water | to 100 |

Example 9

This example illustrates a water-in-oil sunscreen lotion according to the invention. The sunscreen lotion had the following formulation:

| | % w/w |
|---|---|
| 2-Hydroxy-4-dodecanoic acid | 1 |
| Volatile Silicone | 17 |
| Mineral Oil | 1 |
| Butane-1,3-diol | 10 |
| Sodium Chloride | 2 |
| Parsol 1789 (Sunscreen) | 1 |
| Parsol MCX (Sunscreen) | 3 |
| Triethanolamine (to pH 5.5) | qs |
| Demineralised Water | to 100 |

## Claims

1. The use of at least 0.0001% by weight based on the total composition of an hydroxy-organic acid having the structure (1):

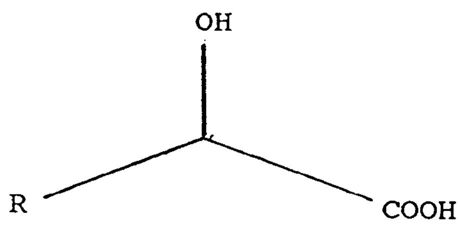

(1)

where
R is chosen from $C_xH_y$ and $C_xH_yOZ$,
x is an integer of from 3 to 16,
y is an integer of from 7 to 33 ,
Z is chosen from -H,R' and $(CH_2)_nOR'$,
R' is chosen from $C_aH_b$ and $(CH_2)_nOC_aH_b$,
a is an integer of from 1 to 4,
b is an integer of from 2 to 9,
n is an integer of from 1 to 6;
and mixtures thereof;
as an inhibitor of microbial spoilage, in a composition intended for topical application to the human body.

2. The use according to claim 1, in which the hydroxyorganic acid is 2-hydroxy-n-octanoic acid.

3. The use according to claim 1, in which the hydroxy organic acid is a 2-hydroxyalkanoic acid chosen from:
2-hydroxy-n-butanoic acid
2-hydroxy-n-hexanoic acid
2-hydroxy-n-octanoic acid
2-hydroxy-n-decanoic acid
2-hydroxy-n-dodecanoic acid
2-hydroxy-n-tetradecanoic acid, and
2-hydroxy-n-hexadecanoic acid.

4. The use according to claim 1, in which the hydroxyorganic acid is a 2-hydroxyalkenoic acid chosen from:
2-hydroxy-4-hexenoic acid

2-hydroxy-4-octenoic acid
2-hydroxy-4-decenoic acid
2-hydroxy-4-dodecenoic acid
2-hydroxy-4-tetradecenoic acid, and
2-hydroxy-4-hexadecenoic acid

**5.** The use according to claim 1, in which the hydroxy organic acid is a 2-hydroxyalkanoic acid chosen from:
2-hydroxy-4,7-octadienoic acid
2-hydroxy-4,9-decadienoic acid
2-hydroxy-4,10-undecadienoic acid
2-hydroxy-4,11-dodecadienoic acid
2-hydroxy-4,13-tetradecadienoic acid, and
2-hydroxy-4,15-hexadecadienoic acid.

**6.** The use according to claim 1, in which the hydroxyorganic acid is a 2-hydroxyalkenoic acid chosen from:
2-hydroxy-6-oxa-4-heptenoic acid
2-hydroxy-7-oxa-4-octenoic acid
2-hydroxy-9-oxa-4-decenoic acid
2-hydroxy-9-oxa-4-undecenoic acid
2-hydroxy-11-oxa-4-dodecenoic acid
2-hydroxy-13-oxa-4-tetradecenoic acid
2-hydroxy-13-oxa-4-pentadecenoic acid
2-hydroxy-14-oxa-4-pentadecenoic acid
2-hydroxy-13-oxa-14-methyl-4-pentadecenoic acid
2-hydroxy-11,14-dioxa-4-pentadecenoic acid
2-hydroxy-13-oxa-4-hexadecenoic acid
2-hydroxy-14-oxa-4-hexadecenoic acid, and
2-hydroxy-15-oxa-4-hexadecenoic acid.

**7.** The use according to any one of the preceding claims wherein the composition contains at least 50% by weight of a cosmetically acceptable vehicle.

European Patent Office

# EUROPEAN SEARCH REPORT

Application Number

EP 93 30 3264

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | EP-A-0 086 070 (UNILEVER NV)<br>* page 10, line 23 - page 13, line 14 *<br>* page 20, line 20 - page 24, line 14; claims 1-32 * | 1-3,7 | A61K7/48<br>A61K47/12 |
| Y | DE-A-2 312 280 (HENKEL & CIE GMBH)<br>* page 16 - page 17; claims 1-6 * | 1-7 | |
| Y | EP-A-0 442 708 (UNILEVER PLC ET AL)<br>* claims 1-13; examples 6-18 * | 1,4-7 | |
| A | EP-A-0 273 202 (E. J. VAN SCOTT ET AL)<br>* claims 1-5,15-18 * | 1-7 | |
| A | US-A-4 287 214 (EUGENE J. VAN SCOTT ET AL.)<br>* claims 1-24 * | 1-7 | |

TECHNICAL FIELDS SEARCHED (Int. Cl.5 )

A61K

The present search report has been drawn up for all claims

| Place of search | Date of completion of the search | Examiner |
|---|---|---|
| BERLIN | 05 AUGUST 1993 | SIATOU E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)